# FASCICULE DE BREVET EUROPEEN

(11) **EP 4 256 308 B1**
(45) Date de publication et mention de la délivrance du brevet: **05.11.2025**
(21) Numéro de dépôt: 21847983.0
(22) Date de dépôt: 02.12.2021
(51) Int. Cl.: G01N 21/55, A61B 5/00, G01N 21/84, G01N 21/57, G01N 21/47

(54) **DISPOSITIF DE DÉTERMINATION DE LA BRILLANCE RELATIVE D'UNE PLURALITÉ DE FIBRES CAPILLAIRES**
VORRICHTUNG ZUR BESTIMMUNG DES RELATIVEN GLANZES EINER VIELZAHL KAPILLARER FASERN
DEVICE FOR DETERMINING THE RELATIVE SHEEN OF A PLURALITY OF CAPILLARY FIBERS

(30) Priorité: 07.12.2020 FR 2012781
(43) Date de publication de la demande: 11.10.2023
(73) Titulaire: SEB S.A., 69130 Ecully (FR)
(72) Inventeur: PLANTEROSE, Thierry, 69134 ECULLY CEDEX (FR); GHECHOUA, Karim, 69134 ECULLY CEDEX (FR); SABATTIER, Johan, 69134 ECULLY CEDEX (FR)
(74) Mandataire: SEB Développement
(86) Numéro de dépôt international: PCT/FR2021/052181
(87) Numéro de publication internationale: WO 2022/123150

(56) Documents cités:
- EP-A1- 2 693 199
- WO-A1-02/18919
- JP-B2- 3 641 370
- US-A1- 2010 328 650
- US-A1- 2012 043 476
- REICH C ET AL: "Light scattering and shine measurements of human hair : a sensitive probe of the hair surface", vol. 44, no. 4, 1 January 1993 (1993-01-01), US, pages 221 - 234, XP055831902, ISSN: 0037-9832, Retrieved from the Internet <URL:https://citeseerx.ist.psu.edu/viewdoc/download?doi=10.1.1.551.8045&rep=rep1&type=pdf> [retrieved on 20210811]
- NOWBUTH K ET AL: "HAIR SHINE MEASUREMENT (SAMBA HAIR) : EVALUATION OF A HAIR COLOR TREATMENT FOLLOWED SHAMPOOS MULTI-APPLICATION Report N° 320000RAE001-A Version Date Modifications Description Operator Writer Approving 001-A 21/07/17", REPORT N° 320000RAE001-A, 23 November 2017 (2017-11-23), XP055831853, Retrieved from the Internet <URL:bossanovavision.com/wp-content/uploads/Presentations/SAMBA%20Hair%20-%20Shine%20report%20sample.pdf> [retrieved on 20210812]

## Description

### Domaine de l'invention

La présente invention se rapporte à un élément optique pour un dispositif de détermination de la brillance relative d'une pluralité de fibres capillaires et le dispositif associé.

### Art antérieur

De manière connue, le document C. Reich et al., "Light scattering and shine measurements of human hair: A sensitive probe of the hair surface", J. Soc. Cosmet. Chem., 44, 221-234 (1993), divulgue une méthode de mesure de la brillance des cheveux, consistant à déterminer l'intensité de la lumière diffusée à différents angles à l'aide d'un goniophotomètre.

Le document JP-3641370 B2 divulgue un instrument permettant de mesurer la brillance des cheveux en les illuminant à l'aide d'une source lumineuse, et en détectant la lumière réfléchie à l'aide de deux photodétecteurs : l'un pour la lumière réfléchie spéculairement, l'autre pour la lumière réfléchie diffusément.

Le document Nowbuth et al., "Hair shine measurement (Samba hair): Evaluation of a hair color treatment followed shampoos multi-application", Report N° 320000RAE001-A (2017), divulgue une méthode de mesure de la brillance des cheveux utilisant de la lumière polarisée afin de séparer la lumière diffuse de la lumière spéculaire.

Le document FR2950695 décrit une méthode pour la mesure de l'apparence visuelle de fibres biréfringentes arrangés de façon aléatoire et régulière. La méthode comprend l'émission de lumière, la polarisation de la lumière, l'éclairage de fibre à partir de la lumière polarisée, la génération de composantes de réflexion et enfin l'observation desdites composantes de réflexion.

Toutefois, ces solutions ne donnent pas une entière satisfaction.

En effet, la solution présentée dans le document FR2950695 présente un encombrement important et ne permet pas un usage domestique.

La présente invention a pour but de résoudre tout ou partie des inconvénients mentionnés ci-dessus.

### Exposé de l'invention

A cet effet, la présente invention concerne un dispositif de détermination de la brillance relative d'une pluralité de fibres capillaires, ou plus particulièrement d'une mèche de cheveux, dans lequel le dispositif de détermination comprend un dispositif de mesure comportant :
- une unité d'émission de lumière configurée pour émettre un faisceau lumineux de mesure ;
- un élément optique; et
- une unité de mesure de réflexion spéculaire configurée pour mesurer un flux spéculaire issu de la deuxième surface optique d'émergence de l'élément optique, et
- une unité de mesure de réflexion diffuse configurée pour mesurer un flux diffus issu de la deuxième surface optique d'émergence, et
- une unite de calcul agencée pour calculer un ratio entre un flux spéculaire mesuré issu de la réflexion spéculaire et un flux diffus mesuré issu de la réflexion diffuse.

L'élément optique comprend un corps réalisé dans un matériau isotrope et définit une zone de mesure disposée à l'extérieur du corps et formant une surface de mesure de l'élément optique, ledit élément optique comprenant :
- une première surface optique incidente du corps, au travers laquelle un faisceau lumineux de mesure émis par une unité d'émission de lumière est apte à passer pour pénétrer dans le corps de l'élément optique ;
- une première surface optique d'émergence du corps, au travers laquelle le faisceau lumineux de mesure en provenance de la première surface optique incidente est apte à passer pour sortir du corps de l'élément optique, et dans laquelle la première surface optique d'émergence est configurée pour que le faisceau lumineux de mesure émerge en direction de la zone de mesure configurée pour recevoir la pluralité de fibres capillaires ou plus particulièrement une mèche de cheveux ;
- une deuxième surface optique incidente du corps, au travers laquelle une réflexion diffuse et une réflexion spéculaire générées lorsque la pluralité de fibres capillaires est placée dans la zone de mesure et plus particulièrement au niveau de la surface de mesure, sont aptes à passer pour pénétrer dans le corps ; et
- une deuxième surface optique d'émergence du corps, au travers laquelle la réflexion diffuse et la réflexion spéculaire sont aptes à passer pour sortir du corps de l'élément optique. L'élément optique est configuré pour séparer un premier milieu comprenant la zone de mesure d'un deuxième milieu comprenant l'unité d'émission de lumière ainsi que l'unité de mesure de la réflexion spéculaire et de la réflexion diffuse. Avantageusement, une telle disposition permet de s'assurer de la précision de la mesure de brillance relative d'une pluralité de fibres capillaires.

Au sens de la présente invention, le terme « apte à passer au travers » signifie qu'au moins une partie du faisceau passe au travers la surface déterminée.

Avantageusement, une telle disposition permet d'isoler de manière étanche l'unité de mesure de la réflexion spéculaire et de la réflexion diffuse du milieu comprenant la zone de mesure. En d'autres termes, grâce à ces dispositions, le dispositif de mesure peut être intégré dans un volume étanche destiné à évoluer dans un milieu humide, par exemple une brosse à cheveux utilisé généralement dans une salle de bain.

Par le biais de l'élément optique le faisceau lumineux de mesure issu de la première surface optique incidente est ainsi configuré pour être réfléchi par les fibres capillaires reçues dans la zone de mesure en une réflexion diffuse et une réflexion spéculaire. Par ailleurs, plus la réflexion spéculaire est importante et plus la brillance des fibres capillaires est importante. A l'inverse, plus la réflexion diffuse est importante et moins la brillance des fibres capillaires est importante.

Avantageusement, une telle disposition permet également d'obtenir un dispositif de détermination de la brillance relative de fibres capillaires compact et pouvant être intégrée dans un appareil de coiffure par exemple.

Selon un mode de réalisation, le corps de l'élément optique est en Polyméthacrylate de méthyle autrement désigné par l'acronyme PMMA.

Selon un mode de réalisation, le corps comprend un évidement formant un prisme et présentant une surface optique de réflexion configurée pour réfléchir le faisceau lumineux de mesure en provenance de l'unité d'émission de lumière en direction de la première surface optique d'émergence.

Avantageusement, une telle disposition permet de rediriger le flux lumineux de la source lumineuse vers la zone de mesure tout en limitant l'encombrement du dispositif de détermination de la brillance relative de fibres capillaires. En effet, l'unité d'émission de lumière émettant le faisceau lumineux de mesure en direction non pas de la première surface optique d'émergence mais en direction de la surface optique de réflexion permet d'agencer l'unité d'émission de lumière de manière à obtenir un gain de place.

Selon un mode de réalisation, la première surface optique d'émergence est configurée pour que le faisceau lumineux de mesure qui le traverse forme un angle sensiblement perpendiculaire avec sa surface.

Au sens de la présente invention, le terme « sensiblement perpendiculaire » signifie « perpendiculaire ou perpendiculaire à 5° près ».

Avantageusement, une telle disposition permet de limiter les réflexions parasites et permet ainsi de conserver la majorité du flux sur la zone de mesure, et ainsi d'améliorer la précision de la mesure de la brillance relative d'une pluralité de fibres capillaires ou plus particulièrement d'une mèche de cheveux.

Selon un mode de réalisation, la surface de mesure est définie sur un plan formant un angle sensiblement non nul avec le faisceau lumineux de mesure issu de la première surface optique émergente.

Au sens de la présente invention, le terme « sensiblement non nul » signifie « non nul ou non nul à 5° près ».

Selon un mode de réalisation, le plan de la surface de mesure forme un angle sensiblement égal à 45° avec le faisceau lumineux de mesure issu de la première surface optique émergente. Une telle disposition permet d'obtenir une bonne réflexion du faisceau lumineux de mesure en une réflexion diffuse et une réflexion spéculaire. Au sens de la présente invention, le terme « sensiblement égale à 45° » signifie « égale à 45° ou égale à 45° à 5° près ».

Selon un mode de réalisation, la première surface optique d'émergence forme un angle sensiblement perpendiculaire avec la deuxième surface optique incidente.

Au sens de la présente invention, le terme « sensiblement perpendiculaire » signifie « perpendiculaire ou sensiblement perpendiculaire à 5° près ».

Une telle disposition permet de limiter les réflexions parasite tout en conservant la majorité du flux issu de la zone de mesure, et permet ainsi d'améliorer la précision de la mesure de la brillance relative d'une pluralité de fibres capillaire ou plus particulièrement d'une mèche de cheveux.

Selon un mode de réalisation, l'élément optique comprend une première unité de collimation distincte du corps et disposée en amont de la première surface optique incidente et configurée pour réaliser une collimation du faisceau lumineux de mesure.

Avantageusement, une telle disposition permet d'obtenir un faisceau lumineux de mesure orienté avec précision.

Selon une alternative, l'élément optique comprend une première unité de collimation formée par la première surface optique incidente et configurée pour réaliser une collimation du faisceau lumineux de mesure.

Avantageusement, une telle disposition permet d'obtenir un dispositif de mesure ayant un faible encombrement.

Selon un mode de réalisation, afin d'obtenir une première surface optique incidente configurée pour réaliser une collimation, ladite première surface optique incidente présente une courbure convexe.

Selon un autre mode de réalisation, dans lequel la deuxième surface optique d'émergence présente une courbure convexe de manière à ce que la réflexion diffuse et la réflexion spéculaire arrivent sensiblement perpendiculairement à cette deuxième surface optique d'émergence.

Une telle disposition permet d'éviter toute réflexion de la réflexion diffuse et de la réflexion spéculaire. Cela permet donc à l'unité de mesure de réflexion diffuse et à l'unité de mesure de réflexion spéculaire d'obtenir respectivement une mesure optimisée et ainsi d'obtenir une meilleure mesure de la brillance relative d'une pluralité de fibres capillaires, ou plus particulièrement d'une mèche de cheveux.

Selon un mode de réalisation, l'élément optique comprend une unité de filtrage de lumière disposée entre la première surface optique incidente et la première surface optique d'émergence.

Selon un autre mode de réalisation, l'unité de filtrage de lumière comprend un premier diaphragme et un deuxième diaphragme, la combinaison du premier diaphragme et du deuxième diaphragme étant agencée pour réaliser un filtrage du faisceau lumineux selon une composante horizontale et verticale.

Selon un mode de réalisation, le dispositif de détermination de la brillance relative d'une pluralité de fibres capillaires comprend une coque de protection configurée pour recevoir l'élément optique.

Selon un mode de réalisation, la coque de protection est configurée pour isoler hermétiquement du milieu extérieur tous les composants de l'élément optique hormis la zone de mesure et la surface du corps de l'élément optique dans laquelle s'inscrit la surface de mesure.

Cette disposition permet de protéger les composants de l'élément optique des impuretés de la zone de mesure. Au sens de la présente invention, les impuretés peuvent être de toutes sortes telles que des poussières, ou des micros-particules par exemple.

En d'autres termes, la coque et l'élément optique séparent un milieu intérieur comprenant l'unité d'émission de lumière, l'unité de mesure de la réflexion spéculaire et l'unité de mesure de la réflexion diffuse d'un milieu extérieur disposé en regard de la zone de mesure.

Selon un mode de réalisation, l'unité de mesure de réflexion diffuse et l'unité de mesure de réflexion spéculaire sont disposées sur un même plan.

Une telle disposition permet de limiter l'encombrement du dispositif de mesure.

Selon un mode de réalisation, l'unité de mesure de réflexion diffuse est configurée pour mesurer la réflexion diffuse issue de la deuxième surface optique d'émergence présentant un angle sensiblement égal à 35° avec une normale à la surface de mesure.

Au sens de la présente invention, le terme « sensiblement égal » signifie « égale ou égale à 5° près ».

Selon un mode de réalisation, il existe une pluralité de réflexions diffuses, chacun présentant un angle différent avec la normale à la surface de mesure. Mesurer la réflexion diffuse présentant un angle sensiblement égal à 35° permet d'obtenir une mesure précise dudit flux diffus.

Selon un mode de réalisation, l'unité de mesure de réflexion spéculaire est configurée pour mesurer la réflexion spéculaire issue de la deuxième surface optique d'émergence présentant un angle sensiblement égal à 51° avec la normale à la surface de mesure.

Selon un mode de réalisation, l'unité d'émission de lumière est agencée pour former un faisceau lumineux de mesure de section rectangulaire dont la longueur est orientée selon la largeur du corps de l'élément optique.

Selon un mode de réalisation, la largeur de la section rectangulaire du faisceau lumineux de mesure est inférieure à 4 mm.

Selon un mode de réalisation, le diaphragme parmi le premier diaphragme et/ou le deuxième diaphragme agencé pour réaliser le filtrage du faisceau lumineux selon la composante verticale présente une hauteur d'ouverture inférieure à la largeur de la section rectangulaire du faisceau lumineux de mesure.

Selon un mode de réalisation, l'unité de mesure de réflexion diffuse et l'unité de mesure de réflexion spéculaire sont optiquement isolées de l'environnement extérieur.

Selon un mode de réalisation, l'unité d'émission de lumière est une diode électroluminescente ou une diode laser à cavité verticale émettant par la surface autrement désignée par l'acronyme anglophone VCSEL.

Selon un mode de réalisation, l'unité d'émission est orientée pour que le faisceau lumineux définisse directement un angle sensiblement perpendiculaire avec la première surface optique d'émergence.

Selon un mode de réalisation, l'unité d'émission est orientée pour que le faisceau lumineux définisse un angle sensiblement perpendiculaire avec la première surface optique d'émergence après qu'il ait été réfléchi par la surface optique de réflexion.

Avantageusement, une telle disposition permet de limiter les flux parasites et ainsi d'améliorer la mesure de la brillance relative d'une pluralité de fibres capillaires, ou plus particulièrement d'une mèche de cheveux.

Selon un mode de réalisation l'unité de filtrage est optiquement disposée entre la première surface optique incidente et la première surface optique d'émergence.

Selon un mode de réalisation l'unité de filtrage est optiquement disposée entre la première surface optique incidente et la première surface optique de réflexion.

Selon un mode de réalisation, les surfaces extérieures du corps de l'élément optique sont rendues opaques, à l'exception de la première surface optique incidente, de la deuxième surface optique incidente, de la première surface optique d'émergence et de la deuxième surface optique d'émergence.

Au sens de la présente invention, le terme « opaque » signifie « opaque ou laissant passer 10% de la lumière ».

Avantageusement, une telle disposition permet de limiter les flux parasites et ainsi d'améliorer la mesure de la brillance relative d'une pluralité de fibres capillaires, ou plus particulièrement d'une mèche de cheveux.

Selon un mode de réalisation, les surfaces du corps de l'élément optique est opacifié par sérigraphie noire.

Selon un mode de réalisation, le premier diaphragme et le deuxième diaphragme sont formés par surmoulage de matière sur le corps de l'élément optique.

L'invention s'applique à un appareil de coiffure comprenant un dispositif de détermination de la brillance relative d'une pluralité de fibres capillaires tel que décrit précédemment.

Les différents aspects définis ci-dessus non incompatibles peuvent être combinés.

### Brève description des figures

L'invention sera encore mieux comprise à l'aide de la description détaillée qui est exposée ci-dessous en regard des dessins annexés dans lesquels :
[Fig. 1] La Figure 1 représente une vue schématique illustrant le principe de fonctionnement d'un dispositif de détermination de la brillance relative d'une pluralité de fibres capillaires conformément à la présente invention ;
[Fig. 2] La Figure 2 représente une vue d'un élément optique selon un premier mode de réalisation conformément à la présente invention ;
[Fig. 3] La Figure 3 représente une vue en coupe de l'élément optique de la figure 2.
[Fig. 4] La Figure 4 représente une vue schématique d'un dispositif de détermination de la brillance relative d'une pluralité de fibres capillaires selon un deuxième mode de réalisation conformément à la présente invention.

### Description en référence aux figures

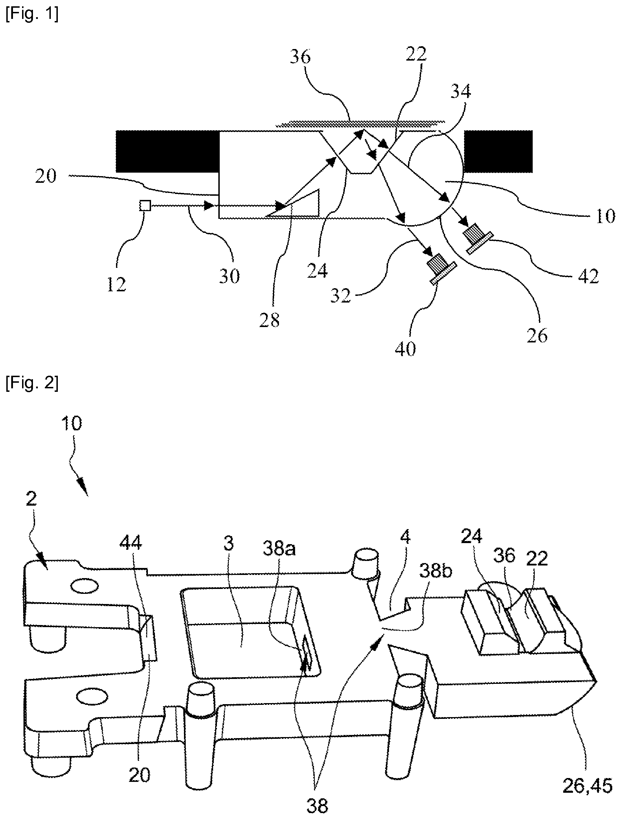

La figure 1 représente une vue schématique illustrant le principe de fonctionnement d'un dispositif de détermination de la brillance relative d'une pluralité de fibres capillaires comprenant notamment un élément optique 10 pour un dispositif de détermination de la brillance relative d'une pluralité de fibres capillaires, ou plus particulièrement d'une mèche de cheveux, configuré pour séparer un premier milieu comprenant une zone de mesure 36 où sont placés la pluralité de fibres capillaires, d'un deuxième milieu contenant une unité d'émission de lumière 12 et des unités de mesure de lumière 40, 42. .

L'élément optique 10, comprend un corps 2 réalisé dans un matériau isotrope translucide, par exemple en polyméthacrylate de méthyle autrement désigné par l'acronyme PMMA, définissant la zone de mesure 36 disposée à l'extérieur du corps 2.

L'élément optique comprend une première surface optique incidente 20 du corps 2, au travers laquelle un faisceau lumineux de mesure 30 émis par une unité d'émission de lumière 12 est apte à passer pour pénétrer dans le corps 2 de l'élément optique 10. L'unité d'émission de lumière 12 peut être une diode électroluminescente ou une diode laser à cavité verticale émettant par la surface autrement désignée par l'acronyme anglophone VCSEL.

Cette unité d'émission de lumière 12 est agencée pour former un faisceau lumineux de mesure 30 de section rectangulaire dont la longueur est orientée selon la largeur du corps 2 de l'élément optique 10.

La largeur de la section rectangulaire du faisceau lumineux de mesure 30 est inférieur à 4 mm.

Comme illustré sur la vue schématique de la figure 1, le corps 2 de l'élément optique 10 comprend un évidement formant une surface optique de réflexion 28 configurée pour réfléchir le faisceau lumineux de mesure 30 en provenance de l'unité d'émission de lumière 12 en direction d'une première surface optique d'émergence 24.

Ainsi, l'unité d'émission de la lumière 12 et la surface optique de réflexion 28 sont orientées pour que le faisceau lumineux de mesure 30, après qu'il ait été réfléchi par la surface optique de réflexion 28, définisse un angle sensiblement perpendiculaire avec la première surface optique d'émergence 24. Avantageusement, une telle disposition permet de rediriger le faisceau lumineux de l'unité d'émission de lumière 12 vers la zone de mesure 36 tout en limitant l'encombrement du dispositif de détermination de la brillance de fibres capillaires. En effet, l'unité d'émission de lumière 12 émettant le faisceau lumineux de mesure 30 en direction non pas de la première surface optique d'émergence 24 mais en direction de la surface optique de réflexion 28, cela permet d'agencer l'unité d'émission de lumière 12 de manière à obtenir un gain de place.

La première surface optique d'émergence 24 est configurée pour que le faisceau lumineux de mesure 30 émerge selon un angle sensiblement perpendiculaire avec sa surface en direction de la zone de mesure 36 configurée pour recevoir la pluralité de fibres capillaires ou plus particulièrement une mèche de cheveux. Au sens de la présente invention, le terme « sensiblement perpendiculaire » signifie « perpendiculaire ou perpendiculaire à 5° près ». Avantageusement, une telle disposition permet de limiter les réflexions parasite et permet ainsi d'améliorer la précision de la mesure de la brillance relative d'une pluralité de fibres capillaires ou plus particulièrement d'une mèche de cheveux.

Selon un mode de réalisation non illustré, l'unité d'émission 12 est orientée pour que le faisceau lumineux de mesure 30 en sortie de l'unité d'émission 12, définisse directement un angle sensiblement perpendiculaire avec la première surface optique d'émergence 24. Un tel mode de réalisation permet de s'affranchir de la surface optique de réflexion 28.

La première surface optique d'émergence 24 forme un angle sensiblement perpendiculaire avec une deuxième surface optique incidente 22 sur lequel est réorienté une partie du faisceau lumineux de mesure 30 après avoir atteint la pluralité de fibres capillaires, ou plus particulièrement la mèche de cheveux disposée sur la zone de mesure 36. Cette disposition de la deuxième surface optique incidente 22 par rapport à la première surface optique d'émergence 24 permet de limiter les réflexions parasites tout en conservant la majorité du flux issu de la zone de mesure 36 en partance pour la deuxième surface optique incidente 22, et permet ainsi d'améliorer la précision de la mesure de la brillance relative d'une pluralité de fibres capillaire ou plus particulièrement d'une mèche de cheveux.

Le flux lumineux de mesure 30 issu de la zone de mesure 36 se décompose en une réflexion diffuse 32 et en une réflexion spéculaire 34 générées lorsque la pluralité de fibres capillaires est placée dans la zone de mesure 36 et plus particulièrement au niveau de la surface de mesure. Plus la réflexion spéculaire est importante et plus la brillance des fibres capillaires est importante. A l'inverse, plus la réflexion diffuse est importante et moins la brillance des fibres capillaires est importante. Avantageusement, une telle disposition permet d'obtenir un dispositif de mesure compact pouvant être intégré dans un instrument de coiffure par exemple.

L'élément optique 10 comprend également une deuxième surface optique d'émergence 26 du corps 2, à travers laquelle la réflexion diffuse 32 et la réflexion spéculaire 34 sont aptes à passer pour sortir du corps 2 de l'élément optique. Au sens de la présente invention, le terme « apte à passer au travers » signifie qu'au moins une partie du faisceau passe à travers la surface déterminée.

La deuxième surface optique incidente 22 peut également présenter une surface concave de manière à réorienter la partie du faisceau lumineux de mesure 30 issue de la zone de mesure 36 vers cette deuxième surface optique d'émergence 26 du corps 2.

Une telle configuration permet donc à l'élément optique 10 de séparer le premier milieu comprenant la zone de mesure 36 du deuxième milieu comprenant l'unité d'émission de lumière 12 ainsi que l'unité de mesure de réflexion spéculaire 42 et de réflexion diffuse 40. Avantageusement, une telle disposition permet d'obtenir un dispositif de détermination de la brillance relative de fibres capillaires dans lequel la zone de mesure 36 et l'unité de mesure de la réflexion spéculaire 42 et de la réflexion diffuse 40 sont séparées par l'élément optique 10. Cela permet d'isoler de manière étanche l'unité de mesure de la réflexion spéculaire 42 et l'unité de mesure de la réflexion diffuse 40 du milieu comprenant la zone de mesure 36. En d'autres termes, grâce à ces dispositions, le dispositif de mesure peut être intégré dans un volume étanche destiné à évoluer dans un milieu humide, par exemple une brosse à cheveux utilisée généralement dans une salle de bain.

L'unité de mesure de réflexion diffuse 40 et l'unité de mesure de réflexion spéculaire 42 sont disposées sur un même plan, ce qui permet de limiter l'encombrement du dispositif de mesure. L'unité de mesure de réflexion diffuse 40 est configurée pour mesurer la réflexion diffuse 32 issue de la deuxième surface optique d'émergence 26 présentant un angle sensiblement égal à 35° avec une normale à la surface de mesure. Au sens de la présente invention, le terme « sensiblement égal » signifie « égale ou égale à 5° près ». Selon un mode de réalisation, il existe une pluralité de réflexions diffuses, chacune présentant un angle différent avec la normale à la surface de mesure. Mesurer la réflexion diffuse présentant un angle sensiblement égal à 35° permet d'obtenir une mesure précise de la réflexion diffuse 32.

L'unité de mesure de réflexion spéculaire 42 est quant à elle configurée pour mesurer la réflexion spéculaire 34 issue de la deuxième surface optique d'émergence 26 présentant un angle sensiblement égal à 51° avec la normale à la surface de mesure. Une telle disposition permet d'obtenir une mesure précise du flux spéculaire.

Selon un mode de réalisation, la deuxième surface optique d'émergence 26 est courbe de manière à ce que la réflexion spéculaire 42 et la réflexion diffuse 40 qui la traverse forment un angle sensiblement perpendiculaire avec ladite surface optique d'émergence 26.

Plus précisément, la deuxième surface optique d'émergence 26 présente une courbure convexe de manière à ce que la réflexion diffuse 32 et la réflexion spéculaire 34 arrivent sensiblement perpendiculairement à cette deuxième surface optique d'émergence 26.

La surface de mesure est définie sur un plan formant un angle sensiblement non nul avec le faisceau lumineux de mesure 30 issu de la première surface optique émergente 24. Au sens de la présente invention, le terme « sensiblement non nul » signifie « non nul ou non nul à 5° près ». Une telle disposition permet au plan de la surface de mesure de former un angle sensiblement égal à 45° avec le faisceau lumineux de mesure 30 issu de la première surface optique émergente 24. Une telle disposition permet d'obtenir une bonne réflexion du faisceau lumineux de mesure 30 en réflexion diffuse et réflexion spéculaire. Au sens de la présente invention, le terme « sensiblement égale à 45° » signifie « égale à 45° ou égale à 45° à 5° près ».

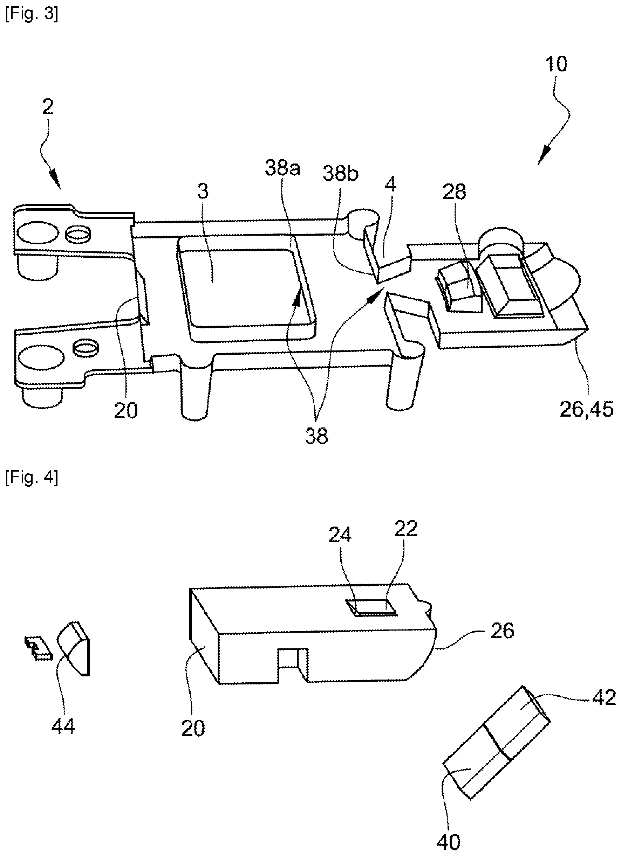

Comme illustré plus particulièrement aux figures 2 et 3, l'élément optique 10 comprend une unité de collimation 44 formée par la première surface optique incidente 20 et configurée pour réaliser une collimation du faisceau lumineux de mesure 30. Avantageusement, une telle disposition permet d'obtenir un élément optique 10 et donc un dispositif de mesure présentant un encombrement davantage limité. Afin d'obtenir une première surface optique incidente 20 configurée pour réaliser une collimation, ladite première surface optique incidente 20 présente une courbure convexe.

Selon une alternative tel que représenté sur la figure 4, le dispositif de détermination de la brillance relative d'une pluralité de fibres capillaires, ou plus particulièrement d'une mèche de cheveux, comprend une unité de collimation 44 distincte du corps 2 et disposée en amont de la première surface optique incidente 20 du corps 2 de l'élément optique 10, et configurée pour réaliser une collimation du faisceau lumineux de mesure 30. Avantageusement, une telle disposition permet d'obtenir un faisceau lumineux de mesure 30 orienté avec précision.

Le corps 2 de l'élément optique 10 forme une unité de filtrage de lumière 38, optiquement disposée entre la première surface optique incidente 20 et la première surface optique d'émergence 24 ; et permet ainsi de limiter les flux parasites et d'améliorer la mesure de la brillance relative d'une pluralité de fibres capillaires, ou plus particulièrement d'une mèche de cheveux.

Plus précisément, dans le mode de réalisation présenté, l'unité de filtrage de lumière 38 est optiquement disposée entre la première surface optique incidente 20 et la première surface optique de réflexion 28.

L'unité de filtrage de lumière 38 comprend un premier diaphragme 38a et un deuxième diaphragme 38b disposés tous deux sur le trajet du faisceau lumineux de mesure 30.

Dans le mode de réalisation présenté, le premier diaphragme est disposé sur un bord d'une cavité 3 ménagé sur le corps 2 de l'élément optique 10.

Ce premier diaphragme 38a présente une hauteur inférieure à la largeur de la section rectangulaire du faisceau lumineux de mesure 30. Ce premier diaphragme 38a élimine ainsi toutes les composantes du faisceau de lumière 30 qui divergeraient trop dans la direction de la hauteur de l'élément optique 10 ou largeur de la section rectangulaire du faisceau de lumière 30.

Le deuxième diaphragme 38b est quant à lui disposé entre le premier diaphragme 38 et la surface de réflexion 28 et est formé par un rétrécissement 4 sur la largeur du corps 2 de l'élément optique 10.

Ce deuxième diaphragme 38b présente une largeur inférieure à la longueur de la section rectangulaire du faisceau de lumière 30. Ce deuxième diaphragme 38b élimine ainsi toutes les composantes du faisceau de lumière 30 qui divergeraient trop dans la direction de la largeur de l'élément optique 10 ou longueur de la section rectangulaire du faisceau de lumière 30.

Aussi, les surfaces extérieures du corps 2 de l'élément optique 10 sont rendues opaques, par application d'une sérigraphie noire, à l'exception de la première surface optique incidente 20, de la deuxième surface optique incidente 22, de la surface de la cavité 3 opposée à la deuxième surface optique incidente 22, de la première surface optique d'émergence 24 et de la deuxième surface optique d'émergence 26.

Au sens de la présente invention, le terme « opaque » signifie « opaque ou laissant passer 10% de la lumière ». Avantageusement, une telle disposition permet de limiter les flux parasites et ainsi d'améliorer la mesure de la brillance relative d'une pluralité de fibres capillaires, ou plus particulièrement d'une mèche de cheveux. Le dispositif de détermination de la brillance relative d'une pluralité de fibres capillaires, ou plus particulièrement d'une mèche de cheveux comprend également une unité de calcul (non illustrée) agencée pour calculer un ratio entre un flux spéculaire mesuré issu de la réflexion spéculaire 42 et un flux réfléchi mesuré issu de la réflexion diffuse 32. Avantageusement, une telle disposition permet de s'assurer de la précision de la mesure de brillance relative d'une pluralité de fibres capillaires.

Le dispositif de détermination de la brillance relative d'une pluralité de fibres capillaires comprend également une pièce de surmoulage opaque (non illustrée) configurée pour recevoir l'élément optique 10. Cette pièce de surmoulage opaque vient isoler optiquement toute la pièce optique 10 à l'exception des zones extérieures au corps 2 de l'élément optique 10 et les surfaces du corps 2 par lesquelles transite le faisceau lumineux de mesure 30.

Ainsi, cette pièce de surmoulage contribue à former le premier diaphragme 38a et le deuxième diaphragme 38b.

La pièce de surmoulage est également utilisée comme support mécanique pour l'élément optique 10 en venant s'insérer en partie dans la cavité 3 et au niveau du rétrécissement 4 du corps 2 de l'élément optique 10.

Cette pièce de surmoulage peut être réalisée en une ou plusieurs parties.

Une coque de protection enveloppant l'élément optique 10 et la pièce de surmoulage est quant à elle configurée pour isoler hermétiquement du milieu extérieur tous les composants de l'élément optique 10 hormis la zone de mesure 36. L'unité de mesure de réflexion diffuse 40 et l'unité de mesure de réflexion spéculaire 42 sont également optiquement isolées de l'environnement extérieur. Cette disposition permet de protéger les composants du dispositif de détermination de la brillance relative d'une pluralité de fibres capillaires, des impuretés de la zone de mesure 36. Au sens de la présente invention, les impuretés peuvent être de toutes sortes telles que des micros-particules par exemple. En d'autres termes, la coque et l'élément optique 10 séparent un milieu intérieur comprenant l'unité d'émission de lumière 12, l'unité de mesure de la réflexion spéculaire 42 et l'unité de mesure de la réflexion diffuse 40 d'un milieu extérieur dans lequel évolue la zone de mesure 36.

Bien entendu, l'invention n'est pas limitée aux modes de réalisation représentés et décrits ci-avant, mais en couvre au contraire toutes les variantes.

## Revendications

1. Dispositif de détermination de la brillance relative d'une pluralité de fibres capillaires, ou plus particulièrement d'une mèche de cheveux, dans lequel le dispositif de détermination comprend un dispositif de mesure comportant :
• une unité d'émission de lumière (12) configurée pour émettre un faisceau lumineux de mesure (30) ;
• un élément optique (10) comprenant un corps (2) réalisé dans un matériau isotrope et définissant une zone de mesure (36) disposée à l'extérieur du corps (2) et formant une surface de mesure de l'élément optique (10), ledit élément optique (10) comprenant :
∘ une première surface optique incidente (20) du corps (2), au travers laquelle un faisceau lumineux de mesure (30) émis par une unité d'émission de lumière (12) est apte à passer pour pénétrer dans le corps (2) de l'élément optique (10);
∘ une première surface optique d'émergence (24) du corps (2), au travers laquelle le faisceau lumineux de mesure (30) en provenance de la première surface optique incidente (20) est apte à passer pour sortir du corps (2) de l'élément optique (10), et dans laquelle la première surface optique d'émergence (24) est configurée pour que le faisceau lumineux de mesure (30) émerge en direction de la zone de mesure (36) configurée pour recevoir la pluralité de fibres capillaires ou plus particulièrement une mèche de cheveux ;
∘ une deuxième surface optique incidente (22) du corps (2), au travers laquelle une réflexion diffuse (32) et une réflexion spéculaire (34) générées lorsque la pluralité de fibres capillaires est placée dans la zone de mesure (36) et plus particulièrement au niveau de la surface de mesure, sont aptes à passer pour pénétrer dans le corps (2) ; et
o une deuxième surface optique d'émergence (26) du corps (2), au travers laquelle la réflexion diffuse (32) et la réflexion spéculaire (34) sont aptes à passer pour sortir du corps (2) de l'élément optique (10) ;
• une unité de mesure de réflexion spéculaire (42) configurée pour mesurer un flux spéculaire issu de la deuxième surface optique d'émergence (26) de l'élément optique (10), et
• une unité de mesure de réflexion diffuse (40) configurée pour mesurer un flux diffus issu de la deuxième surface optique d'émergence (26), et
• une unité de calcul agencée pour calculer un ratio entre un flux spéculaire mesuré issu de la réflexion spéculaire (34) et un flux diffus mesuré issu de la réflexion diffuse (32) ;
l'élément optique (10) étant configuré pour séparer un premier milieu comprenant la zone de mesure (36) d'un deuxième milieu comprenant l'unité d'émission de lumière (12) ainsi que l'unité de mesure de la réflexion spéculaire (42) et l'unité de mesure de la réflexion diffuse (40).

2. Dispositif de détermination de la brillance relative d'une pluralité de fibres capillaires selon la revendication 1, dans lequel le corps (2) comprend un évidement formant un prisme et présentant une surface optique de réflexion (28) configurée pour réfléchir le faisceau lumineux de mesure (30) en provenance de l'unité d'émission de lumière (12) en direction de la première surface optique d'émergence (24).

3. Dispositif de détermination de la brillance relative d'une pluralité de fibres capillaires selon l'une quelconque des revendications 1 à 2, dans lequel la première surface optique d'émergence (24) est configurée pour que le faisceau lumineux de mesure (30) qui le traverse forme un angle sensiblement perpendiculaire avec sa surface.

4. Dispositif de détermination de la brillance relative d'une pluralité de fibres capillaires selon l'une quelconque des revendications 1 à 3, dans lequel la surface de mesure est définie sur un plan formant un angle sensiblement non nul avec le faisceau lumineux de mesure (30) issu de la première surface optique émergente (24).

5. Dispositif de détermination de la brillance relative d'une pluralité de fibres capillaires selon l'une quelconque des revendications 1 à 4, dans lequel la première surface optique d'émergence (24) forme un angle sensiblement perpendiculaire avec la deuxième surface optique incidente (22).

6. Dispositif de détermination de la brillance relative d'une pluralité de fibres capillaires selon l'une quelconque des revendications 1 à 5, comprenant une première unité de collimation (44) soit distincte du corps (2) et disposée en amont de la première surface optique incidente (20) soit formée par la première surface optique incidente (20), et configurée pour réaliser une collimation du faisceau lumineux de mesure (30).

7. Dispositif de détermination de la brillance relative d'une pluralité de fibres capillaires selon l'une quelconque des revendications 1 à 6, dans lequel la deuxième surface optique d'émergence (26) présente une courbure convexe de manière à ce que la réflexion diffuse (32) et la réflexion spéculaire (34) arrivent sensiblement perpendiculairement à cette deuxième surface optique d'émergence (26).

8. Dispositif de détermination de la brillance relative d'une pluralité de fibres capillaires selon l'une quelconque des revendications 1 à 7, comprenant une unité de filtrage de lumière (38) disposée entre la première surface optique incidente (20) et la première surface optique d'émergence (24).

9. Dispositif de détermination de la brillance relative d'une pluralité de fibres capillaires selon la revendication 8, dans lequel l'unité de filtrage de lumière (38) comprend un premier diaphragme (38a) et un deuxième diaphragme (38b), la combinaison du premier diaphragme (38a) et du deuxième diaphragme (38b) étant agencée pour réaliser un filtrage du faisceau lumineux selon une composante horizontale et verticale.

10. Dispositif de détermination de la brillance relative d'une pluralité de fibres capillaires selon l'une des revendications 1 à 9, dans lequel l'unité de mesure de réflexion diffuse (40) et l'unité de mesure de réflexion spéculaire (42) sont disposées sur un même plan.

11. Dispositif de détermination de la brillance relative d'une pluralité de fibres capillaires selon l'une quelconque des revendications 1 à 10, dans lequel l'unité de mesure de réflexion diffuse (40) est configurée pour mesurer la réflexion diffuse (32) issue de la deuxième surface optique d'émergence (26) présentant un angle sensiblement égal à 35° avec une normale à la surface de mesure ; et/ou l'unité de mesure de réflexion spéculaire (42) est configurée pour mesurer la réflexion spéculaire (34) issue de la deuxième surface optique d'émergence (26) présentant un angle sensiblement égal à 51° avec la normale à la surface de mesure.

12. Dispositif de détermination de la brillance relative d'une pluralité de fibres capillaires selon l'une quelconque des revendications 1 à 11, dans lequel l'unité d'émission de lumière (12) est agencée pour former un faisceau lumineux de mesure (30) de section rectangulaire dont la longueur est orientée selon la largeur du corps (2) de l'élément optique (10).

13. Dispositif de détermination de la brillance relative d'une pluralité de fibres capillaires selon les revendications 9 et 12 en combinaison, dans lequel le diaphragme parmi le premier diaphragme (38a) et/ou le deuxième diaphragme (38b) agencé pour réaliser le filtrage du faisceau lumineux selon la composante verticale présente une hauteur d'ouverture inférieure à la largeur de la section rectangulaire du faisceau lumineux de mesure (30).

14. Dispositif de détermination de la brillance relative d'une pluralité de fibres capillaires selon l'une des revendications 12 et 13, dans lequel l'unité de mesure de réflexion diffuse (40) et l'unité de mesure de réflexion spéculaire (42) sont optiquement isolées de l'environnement extérieur.

15. Appareil de coiffure comprenant un dispositif de détermination de la brillance relative d'une pluralité de fibres capillaires selon l'une quelconque des revendications 1 à 14.

## Patentansprüche

1. Vorrichtung zur Bestimmung des relativen Glanzes einer Vielzahl von Haarfasern, oder insbesondere einer Haarsträhne, wobei die Vorrichtung zur Bestimmung eine Messvorrichtung beinhaltet, die Folgendes umfasst:
- eine Lichtemissionseinheit (12), konfiguriert zum Emittieren eines Messlichtstrahls (30);
- ein optisches Element (10), beinhaltend einen Körper (2), der aus einem isotropen Material hergestellt ist und eine Messzone (36) definiert, die an der Außenseite des Körpers (2) angeordnet ist und eine Messoberfläche des optischen Elements (10) bildet, wobei das optische Element (10) Folgendes beinhaltet:
- eine erste optische Einfallsoberfläche (20) des Körpers (2), durch die ein von einer Lichtemissionseinheit (12) emittierter Messlichtstrahl (30) in der Lage ist, zu passieren, um in den Körper (2) des optischen Elements (10) einzudringen;
- eine erste optische Austrittsoberfläche (24) des Körpers (2), durch die der Messlichtstrahl (30) aus der ersten optischen Einfallsoberfläche (20) in der Lage ist, zu passieren, um den Körper (2) des optischen Elements (10) zu verlassen, und wobei die erste optische Austrittsoberfläche (24) konfiguriert ist, damit der Messlichtstrahl (30) in Richtung der Messzone (36) austritt, die konfiguriert ist, um die Vielzahl von Haarfasern, oder insbesondere eine Haarsträhne, aufzunehmen;
- eine zweite optische Einfallsoberfläche (22) des Körpers (2), durch die eine diffuse Reflexion (32) und eine Spiegelreflexion (34), die erzeugt werden, wenn die Vielzahl von Haarfasern in die Messzone (36), und insbesondere auf Höhe der Messoberfläche, platziert wird, in der Lage sind, zu passieren, um in den Körper (2) einzudringen; und
- eine zweite optische Austrittsoberfläche (26) des Körpers (2), durch die die diffuse Reflexion und die Spiegelreflexion (34) in der Lage sind, zu passieren, um den Körper (2) des optischen Elements (10) zu verlassen;
- eine Messeinheit der Spiegelreflexion (42), konfiguriert zum Messen eines Spiegelflusses, der von der zweiten optischen Austrittsoberfläche (26) des optischen Elements (10) stammt, und
- eine Messeinheit der diffusen Reflexion (40), konfiguriert zum Messen eines diffusen Flusses, der von der zweiten optischen Austrittsoberfläche (26) stammt, und
- eine Berechnungseinheit, angebracht zum Berechnen eines Verhältnisses zwischen einem gemessenen Spiegelfluss, der von der Spiegelreflexion (34) stammt, und einem gemessenen diffusen Fluss, der von der diffusen Reflexion (32) stammt;
wobei das optische Element (10) konfiguriert ist, um eine erste Umgebung, die die Messzone (36) beinhaltet, von einer zweiten Umgebung, die die Lichtemissionseinheit (12) sowie die Messeinheit der Spiegelreflexion (42) und der Messeinheit der diffusen Reflexion (40) beinhaltet, zu trennen.

2. Vorrichtung zur Bestimmung des relativen Glanzes einer Vielzahl von Haarfasern nach Anspruch 1, wobei der Körper (2) eine Ausnehmung beinhaltet, die ein Prisma bildet und eine optische Reflexionsoberfläche (28) aufweist, konfiguriert zum Reflektieren des Messlichtstrahls (30) aus der Lichtemissionseinheit (12) in Richtung der ersten optischen Austrittsoberfläche (24).

3. Vorrichtung zur Bestimmung des relativen Glanzes einer Vielzahl von Haarfasern nach einem der Ansprüche 1 bis 2, wobei die erste optische Austrittsoberfläche (24) konfiguriert ist, damit der Messlichtstrahl (30), der sie durchquert, mit ihrer Oberfläche einen im Wesentlichen senkrechten Winkel bildet.

4. Vorrichtung zur Bestimmung des relativen Glanzes einer Vielzahl von Haarfasern nach einem der Ansprüche 1 bis 3, wobei der Messlichtstrahl auf einer Ebene definiert ist, die mit dem Messlichtstrahl (30), der aus der ersten optischen austretenden Oberfläche (24) stammt, einen Winkel von im Wesentlichen nicht null bildet.

5. Vorrichtung zur Bestimmung des relativen Glanzes einer Vielzahl von Haarfasern nach einem der Ansprüche 1 bis 4, wobei die erste optische Austrittsoberfläche (24) mit der zweiten optischen Einfallsoberfläche (22) einen im Wesentlichen senkrechten Winkel bildet.

6. Vorrichtung zur Bestimmung des relativen Glanzes einer Vielzahl von Haarfasern nach einem der Ansprüche 1 bis 5, beinhaltend eine erste Kollimationseinheit (44), die entweder von dem Körper (2) abgetrennt und der ersten optischen Einfallsoberfläche (20) vorgelagert angeordnet ist oder von der ersten optischen Einfallsoberfläche (20) gebildet wird, und konfiguriert ist, um eine Kollimation des Messlichtstrahls (30) durchzuführen.

7. Vorrichtung zur Bestimmung des relativen Glanzes einer Vielzahl von Haarfasern nach einem der Ansprüche 1 bis 6, wobei die zweite optische Austrittsoberfläche (26) eine konvexe Krümmung derart aufweist, dass die diffuse Reflexion (32) und die Spiegelreflexion (34) im Wesentlichen senkrecht an dieser zweiten optischen Einfallsoberfläche (26) ankommen.

8. Vorrichtung zur Bestimmung des relativen Glanzes einer Vielzahl von Haarfasern nach einem der Ansprüche 1 bis 7, beinhaltend eine Lichtfilterungseinheit (38), die zwischen der ersten optischen Einfallsoberfläche (20) und der ersten optischen Austrittsoberfläche (24) angeordnet ist.

9. Vorrichtung zur Bestimmung des relativen Glanzes einer Vielzahl von Haarfasern nach Anspruch 8, wobei die Lichtfilterungseinheit (38) ein erstes Diaphragma (38a) und ein zweites Diaphragma (38b) beinhaltet, wobei die Kombination des ersten Diaphragmas (38a) und des zweiten Diaphragmas (38b) angebracht ist, um eine Filterung des Lichtstrahls gemäß einer horizontalen und vertikalen Komponente durchzuführen.

10. Vorrichtung zur Bestimmung des relativen Glanzes einer Vielzahl von Haarfasern nach einem der Ansprüche 1 bis 9, wobei die Messeinheit der diffusen Reflexion (40) und die Messeinheit der Spiegelreflexion (42) auf einer gleichen Ebene angeordnet sind.

11. Vorrichtung zur Bestimmung des relativen Glanzes einer Vielzahl von Haarfasern nach einem der Ansprüche 1 bis 10, wobei die Messeinheit der diffusen Reflexion (40) konfiguriert ist zum Messen der diffusen Reflexion (32), die von der zweiten optischen Austrittsoberfläche (26) stammt, die mit einer Normalen an der Messoberfläche einen Winkel von im Wesentlichen gleich 35° aufweist; und/oder wobei die Messeinheit der Spiegelreflexion (42) konfiguriert ist zum Messen der Spiegelreflexion (34), die von der zweiten optischen Austrittsoberfläche (26) stammt, die mit einer Normalen an der Messoberfläche einen Winkel von im Wesentlichen gleich 51° aufweist.

12. Vorrichtung zur Bestimmung des relativen Glanzes einer Vielzahl von Haarfasern nach einem der Ansprüche 1 bis 11, wobei die Lichtemissionseinheit (12) angebracht ist zum Bilden eines Messlichtstrahls (30) mit rechteckigem Querschnitt, von dem die Länge gemäß der Breite des Körpers (2) des optischen Elements (10) orientiert ist.

13. Vorrichtung zur Bestimmung des relativen Glanzes einer Vielzahl von Haarfasern nach Ansprüchen 9 bis 12 in Kombination, wobei das Diaphragma unter dem ersten Diaphragma (38a) und/oder dem zweiten Diaphragma (38b), angebracht zum Durchführen der Filterung des Lichtstrahls gemäß der vertikalen Komponente, eine Öffnungshöhe aufweist, die geringer ist als die Breite des rechteckigen Querschnitts des Messlichtstrahls (30).

14. Vorrichtung zur Bestimmung des relativen Glanzes einer Vielzahl von Haarfasern nach einem der Ansprüche 12 und 13, wobei die Messeinheit der diffusen Reflexion (40) und die Messeinheit der Spiegelreflexion (42) von der äußeren Umwelt optisch isoliert sind.

15. Frisiereinrichtung, beinhaltend eine Vorrichtung zur Bestimmung des relativen Glanzes einer Vielzahl von Haarfasern nach einem der Ansprüche 1 bis 14.

## Claims

1. Device for determining the relative sheen of a plurality of hair fibers, or more particularly of a lock of hair, wherein the device for determining comprises a measurement device comprising:
- a light-emitting unit (12) configured to emit a measurement light beam (30);
- an optical element (10) comprising a body (2) made of an isotropic material and defining a measurement region (36) that is placed outside the body (2) and forming a measurement surface of the optical element (10), said optical element (10) comprising:
- a first entrance optical surface (20) of the body (2), through which surface a measurement light beam (30) emitted by a light-emitting unit (12) is able to pass in order to penetrate into the body (2) of the optical element (10);
- a first exit optical surface (24) of the body (2), through which surface the measurement light beam (30) coming from the first entrance optical surface (20) is able to pass in order to exit from the body (2) of the optical element (10), and wherein the first exit optical surface (24) is configured so that the measurement light beam (30) emerges in the direction of the measurement region (36) configured to receive the plurality of hair fibers or more particularly a lock of hair;
- a second entrance optical surface (22) of the body (2), through which surface a diffuse reflection (32) and a specular reflection (34) generated when the plurality of hair fibers is placed in the measurement region (36) and more particularly at the measurement surface, are able to pass in order to penetrate into the body (2); and
- a second exit optical surface (26) of the body (2), through which surface the diffuse reflection (32) and the specular reflection (34) are able to pass to exit from the body (2) of the optical element (10);
- a specular reflection measurement unit (42) configured to measure a specular flow coming from the second exit optical surface (26) of the optical element (10), and
- a diffuse reflection measurement unit (40) configured to measure a diffuse flow coming from the second exit optical surface (26), and
- a calculation unit arranged to calculate a ratio between a measured specular flow coming from the specular reflection (34) and a measured diffuse flow coming from the diffuse reflection (32);
the optical element (10) being configured to separate a first area comprising the measurement region (36) from a second area comprising the light-emitting unit (12) as well as the specular reflection measurement unit (42) and the diffuse reflection measurement unit (40).

2. Device for determining the relative sheen of a plurality of hair fibers according to claim 1, wherein the body (2) comprises a recess forming a prism and having a reflection optical surface (28) configured to reflect the measurement light beam (30) coming from the light-emitting unit (12) in the direction of the first exit optical surface (24).

3. Device for determining the relative sheen of a plurality of hair fibers according to any of claims 1 to 2, wherein the first exit optical surface (24) is configured so that the measurement light beam (30) that passes through it forms a substantially perpendicular angle with its surface.

4. Device for determining the relative sheen of a plurality of hair fibers according to any of claims 1 to 3, wherein the measurement surface is defined on a plane forming a substantially non-zero angle with the measurement light beam (30) coming from the first exit optical surface (24).

5. Device for determining the relative sheen of a plurality of hair fibers according to any of claims 1 to 4, wherein the first exit optical surface (24) forms a substantially perpendicular angle with the second entrance optical surface (22).

6. Device for determining the relative sheen of a plurality of hair fibers according to any of claims 1 to 5, comprising a first collimation unit (44) either separate from the body (2) and arranged upstream of the first entrance optical surface (20) or formed by the first entrance optical surface (20), and configured to perform a collimation of the measurement light beam (30).

7. Device for determining the relative sheen of a plurality of hair fibers according to any of claims 1 to 6, wherein the second exit optical surface (26) has a convex curvature in such a way that the diffuse reflection (32) and the specular reflection (34) arrive substantially perpendicularly to this second exit optical surface (26).

8. Device for determining the relative sheen of a plurality of hair fibers according to any of claims 1 to 7, comprising a light-filtering unit (38) arranged between the first entrance optical surface (20) and the first exit optical surface (24).

9. Device for determining the relative sheen of a plurality of hair fibers according to claim 8, wherein the light-filtering unit (38) comprises a first diaphragm (38a) and a second diaphragm (38b), the combination of the first diaphragm (38a) and of the second diaphragm (38b) being arranged to perform a filtering of the light beam according to a horizontal and vertical component.

10. Device for determining the relative sheen of a plurality of hair fibers according to one of claims 1 to 9, wherein the diffuse reflection measurement unit (40) and the specular reflection measurement unit (42) are arranged on the same plane.

11. Device for determining the relative sheen of a plurality of hair fibers according to any of claims 1 to 10, wherein the diffuse reflection measurement unit (40) is configured to measure the diffuse reflection (32) coming from the second exit optical surface (26) having an angle substantially equal to 35° with a normal to the measurement surface; and/or the specular reflection measurement unit (42) is configured to measure the specular reflection (34) coming from the second exit optical surface (26) having an angle substantially equal to 51° with the normal to the measurement surface.

12. Device for determining the relative sheen of a plurality of hair fibers according to any of claims 1 to 11, wherein the light-emitting unit (12) is arranged to form a measurement light beam (30) of rectangular section the length of which is oriented in the direction of the width of the body (2) of the optical element (10).

13. Device for determining the relative sheen of a plurality of hair fibers according to claims 9 and 12 in combination, wherein the diaphragm among the first diaphragm (38a) and/or the second diaphragm (38b) arranged to perform the filtering of the light beam according to the vertical component has an aperture height less than the width of the rectangular section of the measurement light beam (30).

14. Device for determining the relative sheen of a plurality of hair fibers according to one of claims 12 and 13, wherein the diffuse reflection measurement unit (40) and the specular reflection measurement unit (42) are optically isolated from the outside environment.

15. Hair styling apparatus comprising a device for determining the relative sheen of a plurality of hair fibers according to any of claims 1 to 14.
